# EUROPEAN PATENT APPLICATION

(11) **EP 2 053 396 A1**
(43) Date of publication of application: **29.04.2009**
(21) Application number: 07118964.1
(22) Date of filing: 22.10.2007
(51) Int. Cl.: G01N 33/50

(54) **Device and method for the monitoring of the movement of cells**

(71) Applicant: Koninklijke Philips Electronics N.V., 5621 BA Eindhoven (NL)
(72) Inventor: The designation of the inventor has not yet been filed
(74) Representative: Ledeboer, Johannes Albertus

(57) **Abstract**

The present invention relates to a device for the monitoring of very small movements of cells, in particular when undergoing chemotaxis. The device is based on electrode units (3) comprising a number of electrodes for creating electrical fields surrounding an optical detector (2), e.g. an integrated PIN diode. A gradient of growth factor (stimulant) is obtained in a stimulant gradient chamber (1) with various methods, including the use of microfluidic methods or patterning a hydrogel layer on top of the electronics. The device is capable of measuring very small movements of cells (C) and so is ideally suited to fast sensitive chemotaxis assays.

## Description

### TECHNICAL FIELD OF THE INVENTION

The present invention relates to a device for the monitoring of the movement of cells and to a corresponding method.

### BACKGROUND OF THE INVENTION

The mobility of cells in the human body is of vital importance for various biological processes. Cells which can be stimulated to move, often known as chemokinetic or chemotactic cells, include stem cells, leukocytes, fibrolasts, tumor cells and of course sperm cells. The cells with the highest mobility are neutrophils which can move at 15-20 µm/ min while the slowest are the fibroblasts which move at only 0.2-1 µm/min.

Monocytes, a type of leukocyte which can chemotax, have many important functions within the human body. One of their most important roles is in the immune system where they are involved in the phagocytosis of foreign cells and materials. Another important property of monocytes is their ability to adhere, penetrate and differentiate at the sites of atherosclerotic plaque formation in arteries. Recently a link between monocyte mobility and atherosclerotic risk has been suggested. More specifically, patients with high risk factors for CAD (such as diabetes and smoking) demonstrate a drastic reduction in monocyte mobility, providing a potential CAD risk stratification based upon monocyte chemotaxis.

These effects are described in more detail in
i) Vascular Endothelial Growth Factor-A- Induced Chemotaxis of Monocytes is attenuated in patients with Diabetes Mellitus: A potential predictor for the individual capacity to develop collaterals, Johnnes Waltenberger et al, Circulation 2000;102; P. 185-190;
ii) Hypercholesterolaemia impairs monocyte function in CAD patients, F.S. Czepluch et al, Jour. Of Inter. Medicine 261; P. 201-204; 2007;
iii) N. Stadler et al: Smoking-induced monocyte Dysfunction is reversed by vitamine C supplementation in vivo, Arteriscler. Thromb. Vasc. Biol. 2007;27; P. 120-126.

The traditional method for measuring the mobility of cells such as monocytes is based on a so-called Boyden chamber. Such a device consists of two chambers separated by a thin membrane. The lower chamber is filled with the stimulant (e.g. growth factor), and in the upper chamber the cells are loaded. A chemotactic gradient develops across the thickness of the membrane. After an incubation period the membrane is removed and examined under a microscope to count the number of cells that had sufficient mobility to traverse the membrane. Boydon chamber chemotaxis assays are actually very inefficient assays with only about 10% of the initial sample liquid being used.

The problem with the known method of measuring the cell mobility is three fold. Firstly, it takes a long period of time for the cells to migrate through the membrane thus the assay is time consuming. Secondly, it is labor intensive to count the number of cells present on the membrane. Finally, the Boyden chamber assay only yields the total number of cells that crossed the membrane after a certain time. Cells which migrate through the membrane in the beginning of the incubation time are not distinguishable from cells which migrate later in time.

The movement of cells can particularly be chemotactic, chemokinetic, chemoinvasion or haptotaxis. In chemotaxis the movement of cells is induced by a concentration gradient of a soluble chemotactic stimulus. In haptotaxis the movement of cells is induced by a concentration gradient of a substrate-bound stimulus. In chemoinvasion the movement of cells into/through a barrier or gel is induced by a concentration gradient of a chemotactic stimulus. With chemokinetic the movement of cells happens under a concentration of a chemical but where no gradient is present.

### SUMMARY OF THE INVENTION

It is an object of the present invention to provide a device and method which allow to perform time-resolved measurements of cell migration, e.g., to study the distribution of chemotaxis over a cell population or to study the movement of the same cell under various conditions (i.e. different concentration gradients, after drug treatments etc.).

The object is achieved according to the present invention by a device as defined in claim 1 comprising:
- a stimulant gradient chamber for providing a concentration gradient of a stimulant, said stimulant gradient chamber having a cell inlet for the inlet of cells,
- an optical detector for detecting shadows from cells located between a light source and said detector and for generating a detection signal,
- an electrode unit comprising a number of electrode elements surrounding said optical detector,
- a voltage supply unit for supplying the electrode elements of said electrode unit with drive voltages,
- a signal measurement unit for measuring said detection signal, and
- a control unit for controlling said voltage supply unit based on said measured detection signal.

The object is further achieved by a corresponding method as defined in claim 23.

The device and method of the present invention allow to measure the mobility of thousands of cells simultaneously and the distribution of the mobility. By the invention, the time needed to perform the assay is considerably reduced. The invention is based on the idea to use (large area) electronics including an optical detector and electrodes surrounding said optical detector, which are, preferably, integrated onto a substrate. The present invention uses preferably large area electronics, and the integrated electronics are preferably based on LTPS (low temperature poly-silicon) or amorphous Si. The detection is based on the fact that the shadow from the cell blocks a fraction of illumination light from striking the optical detector. This can be detected by monitoring the output signal of the optical detector.

Preferred embodiments of the invention are defined in the dependent claims. It shall be noted that the claimed method can be developed further in the same or similar way as defined in the dependent claims of the claimed device.

According to a preferred embodiment the control unit is adapted for controlling the voltage supply of an electrode unit such that, in case of a monitored movement of a cell in a direction away from a holding position, the supplied drive voltage is changed to prevent said movement. Preferably, the control unit is adapted for increasing frequency and/or amplitude of the supplied drive voltage in case of a monitored movement of a cell in a direction away from a holding position. This provides a kind of feedback loop where the dielectrophoretic force from the electrical field is being balanced against the force being exerted by the cell due to chemotaxis. Thus, by measuring of either the amplitude or frequency of the drive signal to the electrode elements an information of the chemotactic mobility of the cell is obtained.

The electrode unit is preferably a quadrupole electrode or an octopole electrode as defined in further dependent claims. Neighbouring electrode elements are preferably provided with high frequency drive voltages having a phase difference, in particular of 180°. The generated electric field profile, provided negative dielectrophoresis occurs, results in cells collecting at the central region where the optical detector is placed.

The detector is preferably a light sensitive semiconductor element, in particular PIN diode whose photo-current is monitored. However, other light sensitive elements can be used as detector as well.

Generally, the ambient light can be used for illuminating the stimulant gradient chamber. If there is, however, not sufficient ambient light, or if a certain light intensity or color is required, an extra light source can be provided in addition.

Further, it is preferred to provide a calibration unit, in particular a photodiode, for calibration of said signal measurement unit.

Different advantageous embodiments of the chemotaxis device of the present invention, in particular of the stimulant gradient chamber, are defined in claims 9 to 15.

In order to enable the cells to migrate it is proposed to add an adherence layer, for instance on top of the optical detector and the electrode unit, onto which the cells can adhere.

To allow the monitoring of a large number of cells simultaneously it is proposed not only to provide a single optical detector and a single electrode unit but an array of optical detectors and a plurality of electrode units, each including a number of electrode elements and surrounding one optical detector of said array.

Further, the direction of movement of a cell can be detected if there are at least two optical detectors, for instance four optical detectors, surrounded by the electrode elements of an electrode unit.

### BRIEF DESCRIPTION OF THE DRAWINGS

The invention will now be explained in more detail with reference to the following exemplary non-limiting embodiments. In the following:
Fig. 1 shows a schematic block diagram of a chemotaxis device according to the present invention,
Fig. 2 shows a first embodiment of electrode units including a plurality of quadrupole electrodes,
Fig. 3 shows a second embodiment of electrode units including a plurality of octopole electrodes,
Fig. 4 shows a schematic block diagram of a first embodiment of a stimulant gradient chamber according to the present invention,
Fig. 5 shows a schematic block diagram of a second embodiment of a stimulant gradient chamber according to the present invention,
Fig. 6 shows a schematic block diagram of a third embodiment of a stimulant gradient chamber according to the present invention,
Fig. 7 shows a schematic diagram of a fourth embodiment of a stimulant gradient chamber according to the present invention,
Fig. 8 shows various diagrams illustrating different arrangement of an adherence layer according to the present invention,
Fig. 9 shows a flow chart of an embodiment of the method according to the present invention for direction measurement,
Fig. 10 shows an electrode unit and the direction of the stimulant gradient with respect to said electrode unit,
Fig. 11 shows a segmented optical detector surrounded by the electrodes of an electrode unit,
Fig. 12 shows an optical detector surrounded by ring electrodes,
Fig. 13 shows an optical detector surrounded by segmented ring electrodes,
Fig. 14 shows a schematic diagram of an embodiment using haptotaxis, and
Fig. 15 shows a schematic diagram of an embodiment using chemoinvasion.

### DETAILED DESCRIPTION OF THE INVENTION

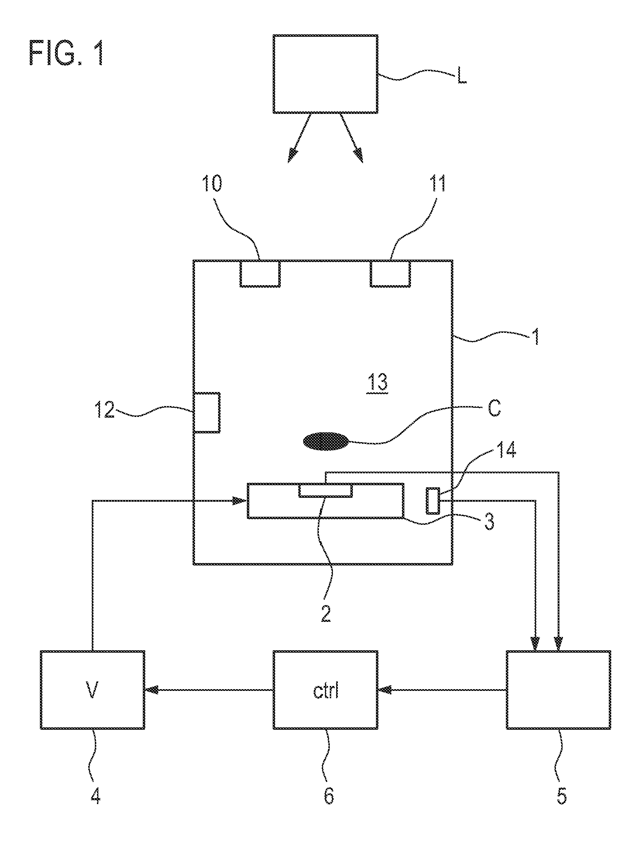

Fig. 1 shows a schematic block diagram of a chemotaxis device according to the present invention including a stimulant gradient chamber 1 for providing a concentration gradient of a stimulant over the areas where the cells C to be monitored are located. To create the concentration gradient, the stimulant gradient chamber 1 generally comprises a stimulant inlet 10 for the inlet of the stimulant (e.g. a growth factor), a buffer inlet 11 for the inlet of a buffer solution and a cell inlet 12 for the inlet of cells to be monitored into the inner volume 13 of the chamber 1. There are various ways of providing a concentration gradient of a stimulant. These will be explained below in connection with various embodiments of the invention.

An optical detector 2 (or, preferably, an array of optical detectors 2) for detecting shadows from cells located between a light source L (which can be included in the chemotaxis device, can be separately/externally provided or can simply be any available source of ambient light) for providing light and the detector 2 and for generating a detection signal is arranged in the inner volume 13. Further, an electrode unit 3 comprising a number of electrode elements surrounding the optical detector 2 (or, preferably, a plurality of electrode units 3 each comprising a number of electrode elements surrounding one or more of the optical detectors 2 of an array of optical detectors) is arranged in the inner volume 13. Both the optical detector 2 and the electrode unit 3 are only schematically shown in Fig. 1, particular embodiments thereof are shown and explained in more detail below.

For supplying the electrode elements said electrode unit 3 with drive voltages a voltage supply unit 4 is provided. The detection signal generated by the optical detector 2 is measured by a signal measurement unit 5. Based on the measured detection signal the voltage supply unit 4 is controlled by a control unit 6.

To enable a calibration of the available elements of the chemotaxis device, in particular of the signal measurement unit 5 with respect to the available light, a calibration unit 14, e.g. a photodiode, is preferably (but not necessarily) provided in addition.

The following embodiments will be explained referring to an array of optical detectors and a plurality of electrode units enabling the simultaneous monitoring of a plurality of cells. However, as mentioned above, a single optical detector and a single electrode unit is generally sufficient for the monitoring of the movement of cells.

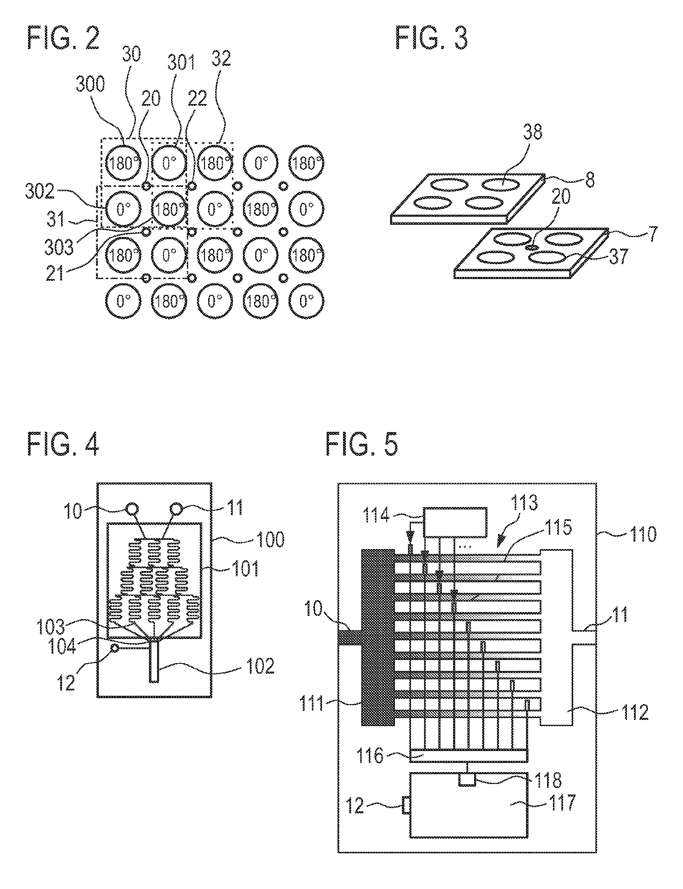

A first embodiment of electrode units including a plurality of quadrupole electrodes 30, 31, 32 is shown in Fig. 2. In this general embodiment an array of optical detectors 20, 21, 22 is shown, each optical detector 20, 21, 22 being surrounded by the electrode elements of a quadrupole electrode 30, 31, 32. For instance, the detector 20 is surrounded by the electrode elements 300, 301, 302, 303 of the quadrupole electrode 30. As becomes clear from Fig. 2, the quadrupole electrodes are - in this embodiment - overlapping, i.e. the electrodes in the center belong to four quadrupole electrodes which provides a high density.

The electrodes are driven by the supply unit 4 with sine wave voltage profiles with frequencies between 50 kHz and 5 MHz, preferably between 100 kHz and 1 MHz with neighbouring electrode elements (e.g. electrode element 300 as the neighbour of electrode elements 301, 302) having a phase difference of preferably 180°. This well-known method of driving quadrupole electrodes 30, 31, 32 generates an electric field profile that, provided negative dielectrophoresis occurs, results in cells collecting at the central regions of the quadrupole electrodes as indicated by the circles 20, 21, 22 in Fig. 2 where optical detectors are located according to the present invention.

Preferably, the optical detectors are semiconductor elements, e.g. PIN diodes, and are integrated in the substrate, which carries also the quadrupole electrodes, at these locations so that the presence of a cell can be detected. Detection is based on the fact that the shadow from the cells (approximately 12% as determined in experiments) blocks a fraction of illumination light from striking the respective optical detector. This can be detected by monitoring the detection signal of the respective optical detector (e.g. the photo-current in case of a PIN diode).

When a stimulant concentration is present in the chamber 1, the cells being held in the electrode units (e.g. quadrupole units 30, 31, 32) will attempt to migrate in the direction of the gradient. During this attempt at migration the detection signals are monitored with the signal measurement unit 5 (e.g. integrated electronic circuits). If the detection signal indicates a movement of a cell away from the holding position (i.e. if the detection signal shows an increase) then the frequency or voltage amplitude of the drive signal supplied to the relevant quadrupole electrode can be increased in order to prevent movement and/or forcing the cell back to the holding position.

In effect this is a feed-back loop where the dielectrophoretic force from the electrical field is being balanced against the force being exerted by the cell due to chemotaxis. Measurement of the amplitude and/or frequency of the drive voltage signal is a measurement of the mobility of the cell.

The optical detectors are approximately the same size as the cell, and thus a movement of only a few microns is detectable. With a small array of only 100 x 100 sites the mobility of 10000 cells can be measured individually. This allows the distribution of mobility to be measured over a large cell population. As shown above the neighbouring electrode elements can be shared by neighbouring electrode units. This, however, does not allow an independent control. Thus, in an alternative embodiment, the electrode elements belong only to one electrode unit and not to several neighbouring electrode units.

While the embodiment shown in Fig. 2 uses quadrupole electrodes as electrode units, it may be preferable to use octopole electrodes as electrode units as illustrated in Fig. 3. In this embodiment the same array pattern of electrodes 38 are fabricated on a top substrate 8 (on the bottom thereof) arranged in parallel to a bottom substrate 7 carrying the pattern of electrodes 37 on its top surface as shown in Fig. 2. As can be seen optical detectors (indicated by one detector 20) are only provided in or on the bottom substrate 7 but not on the top substrate 8. This configuration allows creating a 3D field trap, where the voltage applied on each quadrupole electrode can be adjusted to vary the force with which the cells are "pressed" against the substrate.

Fig. 4 shows a schematic block diagram of a first embodiment of a stimulant gradient chamber according to the present invention. In this embodiment the stimulant gradient chamber 100 includes a branch structure 101 and a monitoring chamber 102. The branch structure 101 has a plurality of interconnected branches for mixing the stimulant and the buffer solution provided via the stimulant inlet 10 and the buffer inlet 11, respectively, into said concentration gradient of said stimulant. Via a concentration gradient outlet 103 the generated concentration gradient is provided to a concentration gradient inlet 104 of the monitoring chamber 102, into which the cells are loaded via the cell inlet 12. Within said monitoring chamber 102 the array 2 of optical detectors and the plurality of electrode units 3 are arranged.

A schematic block diagram of a second embodiment of a stimulant gradient chamber 110 according to the present invention is shown in Fig. 5. In this embodiment the stimulant gradient chamber 110 comprises a stimulant chamber 111 having a stimulant inlet 10 for the inlet of the stimulant, a buffer chamber 112 having a buffer inlet 11 for the inlet of the buffer solution, a plurality of channels 113 connecting said stimulant chamber 111 and said buffer chamber 112, a tapping means 114 for tapping said channels 113 at different tapping locations 115, a concentration gradient outlet 116 for the outlet of the generated concentration gradient, and a monitoring chamber 117 having the cell inlet 12 and a concentration gradient inlet 118 for the inlet of said concentration gradient. Again, within said monitoring chamber 117 the array 2 of optical detectors and the plurality of electrode units 3 are arranged (not shown).

This embodiment is based on the idea to connect two volumes 111, 112 together via many narrow channels 113. By filling one volume 111 with stimulant and the other 112 with buffer a gradient in stimulant concentration is established along the channels 113. By tapping these channels 113 at locations 115 which start near one volume 111 and progressively move towards the other volume 112 it is possible to create a gradient at the outlet 116 where the monitoring chamber 117 is situated.

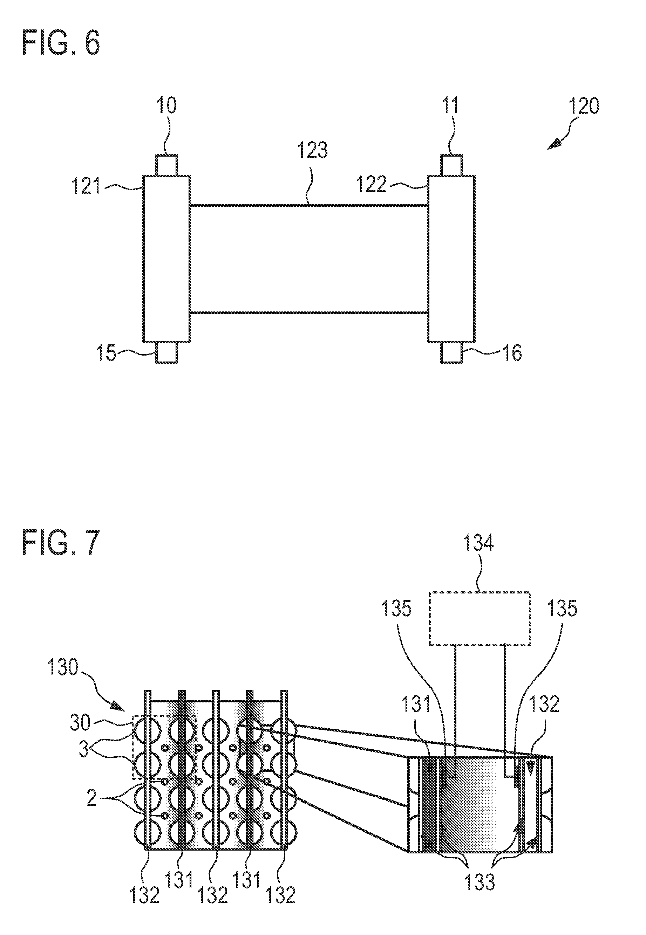

Fig. 6 shows a schematic block diagram of a third embodiment of a stimulant gradient chamber 120 according to the present invention. In this embodiment the stimulant gradient chamber 120 again comprises a stimulant chamber 121 having a stimulant inlet 10 for the inlet of the stimulant, a stimulant outlet 15, and a buffer chamber 122 having a buffer inlet 11 for the inlet of the buffer solution and a buffer outlet 16, which are connected by a diffusion chamber 123 for creating the gradient in stimulant concentration by diffusion of the stimulant and the buffer solution into the diffusion chamber 123. Within said diffusion chamber 123 the array of optical detectors and the plurality of electrode units are arranged (not shown).

Fig. 7 shows a schematic block diagram of a fourth embodiment of a stimulant gradient chamber 130 according to the present invention. In this embodiment a local gradient in stimulant is obtained by defining microfluidic channels 131, 132 on top of the optical detectors 2. The walls 133 of these channels 131, 132 are slightly porous to aqueous solutions. An example of such a material is a gel type material (preferably a hydrogel type material) such as polyacrylamide. Preferably the polyacrylamide would be photosensitive to allow direct photolithography to define the structures.

By filling alternate channels with stimulant and buffer solution, e.g. channels 131 with stimulant and channels 132 with buffer solution, it is possible to create a local gradient as illustrated schematically in Fig. 7. An added advantage of such channels 131, 132 is that it also aids the filling of the array with cells. With a liquid flow along the direction defined by the channels 131, 132 the cells are transported to the quadrupole electrodes 30.

The hydrogel material that can be used in this embodiment can be based on e.g.:
i) Anionic monomers: e.g., (meth)acrylic acid, p-styrene sulfonic acid, itaconic acid (CH2C(COOH)CH2COOH), crotonic acid (CH3CHCHCOOH);
ii) Cationic monomers: e.g., vinyl pyridine, aminoethyl (meth)acrylates, acrylamide;
iii) Neutral monomers: e.g., hydroxyethyl (meth)acrylate and vinyl acetate.

In the embodiment shown in Fig. 7 hydrogel channels 131, 132 are proposed which allows stimulant to passively diffuse into the volume containing the optical detectors 2. This is possible if there is a flow of solutions through the channels 131, 132. However, it may be preferable to avoid the need for flow and create chambers where stimulant and buffer solution are statically present. In this case the volumes will become depleted as the stimulant and buffer solution diffuse away and so the gradient will diminish. It is therefore advantageous to be able to actively control the diffusion into the monitoring chamber.

To enable this, in a further embodiment electrodes 135 are placed under the hydrogel walls and the hydrogel is made responsive to either an applied voltage or an associated pH change. Upon applying a voltage to said control electrodes 135 via a control voltage supply 134 the porosity of the walls 133 can be increased in order to compensate for a depletion of the stimulant.

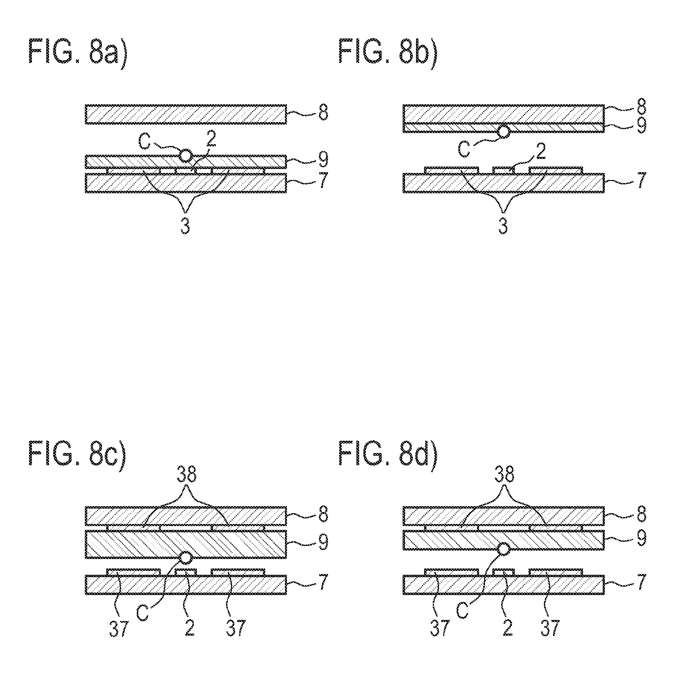

Fig. 8 shows various diagrams illustrating different arrangement of an adherence layer according to the present invention. All cells which undergo chemotaxis are adherent cells. i.e. they adhere onto surfaces. If these cells are suspended at locations between the electrode units without being in contact with a surface then they will not be able to migrate. It is therefore advantageous to cover one of the surfaces with an adherence layer, for instance made of a gel material, e.g. a hydrogel material (for instance the materials mentioned above for the channel walls), onto which the cells can adhere. There are various possibilities where such an adherence layer can be provided

The most simple embodiment is illustrated in Fig. 8a. In this embodiment the electrode unit(s) 3 and the optical detector 2 are placed onto the bottom substrate 7 and are further covered with an adherence layer 9. The top substrate 8 (which can also be left out) is only provided in this embodiment to protect the structure and/or to prevent disturbance of the stimulant gradient, e.g. by an air flow.

If hydrogel is used as material for the adherence layer the hydrogel should have a high water content so that the conductivity is approximately the same as the solution. This ensures that the electrical fields can easily penetrate the sample solution. Further, the thickness of the hydrogel should exceed the levitation height of the cell. The levitation height is the height above the substrate where gravity is balanced by the dielectrophoretic force. By choosing a thickness of the hydrogel greater than the levitation height, the cell is placed in contact with the adherence layer when the electrical field is applied, thereby facilitating cell adhesion. An advantage of covering the electrodes is that it also avoids the possibility of a cell touching an electrode and undergoing electrical lysis.

Other materials rather than hydrogel can be collogen or fibronectin or other protein matrices, as long as they are non-insulating and can be deposited thick enough.

Another embodiment is illustrated in Fig. 8b and involves placing adherence layer 9 on the top substrate 8. This avoids the restriction on the conductivity of the adherence layer 9 and thus allows more freedom in choosing the material. Since the gel used preferably for the adherence layer 9 is often scattering when swollen, placing it on the top substrate 8 is preferable rather than placing it between the cell C and the detector 2. The thickness of the adherence layer 9 should be sufficient so that the electrical field forces the cell into the adherence layer 9.

Further embodiments using octopole electrodes as electrode units, where four electrodes 37, 38 are present on each of the substrates 7, 8 are illustrated in Figs. 8c and 8d. Again the adherence layer 9 can be present either on the top or bottom substrates 7, 8. When a cell is trapped between octopole electrodes then the levitation height will be slightly less than halfway between the two substrates 7, 8 (due to the contribution of gravity). To ensure contact between the adherence layer 9 and the cell C the thickness of the adherence layer 9 should be slightly more than half the spacer distance. It could for example be three quarters of the spacer.

Finally, it is possible to ensure contact between the adherence layer 9 and the cell C by driving the electrodes 37, 38 on opposite substrates 7, 8 with different voltage amplitudes and/or frequencies. This shifts the electrical field minimum away from the centre of the spacer. For example, if the cell C has to be forced up into the adherence layer 9 as is the case in Fig. 8d then the top electrodes 38 could be driven with a lower voltage than the bottom electrodes 37.

In summary, according to the present invention a chemotaxis device is proposed which is designed to measure very small movements of cells when undergoing chemotaxis. The device is based on electrode units comprising a number of electrodes for creating electrical fields surrounding an optical detector, e.g. an integrated PIN diode. A gradient of growth factor (stimulant) is obtained with various methods, including the use of microfluidic methods or patterning a hydrogel layer on top of the electronics. The device is capable of measuring very small movements of cells and so is ideally suited to fast sensitive chemotaxis assays.

The movement of a cell is preferably detected by supplying drive voltages having a high frequency and/or high voltage amplitude to said electrode elements thus making the cell move to the position of the detector (which, generally, is the middle of the electrode unit). Thereafter, the voltage amplitude and/or frequency of said drive voltages is reduced until movement of the cell is detected, which is indicated by an increase of the detector signal from the optical detector.

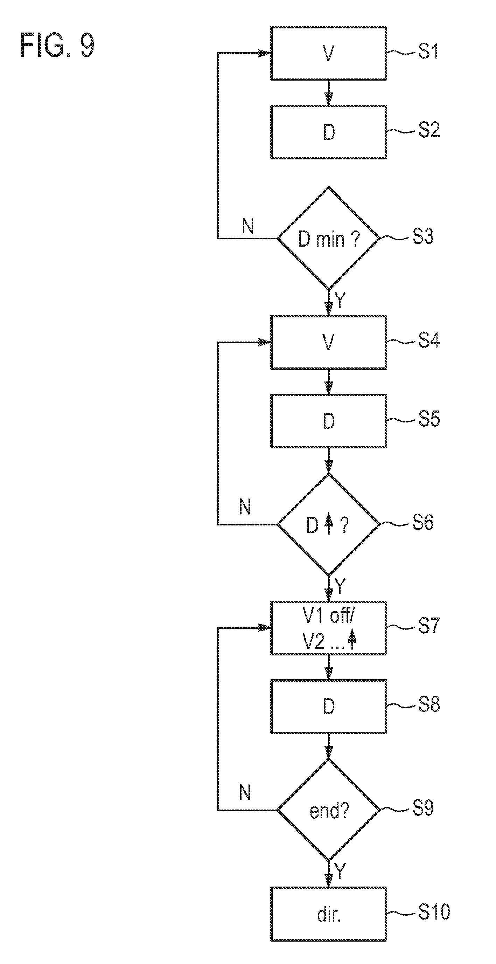

The directionality of the movement of a cell can, for instance, be detected by the steps as shown in the flow chart of Fig. 9. In step S1 the same drive voltages V are applied to all electrode elements of the electrode unit to move the cell to the position of the detector. In step S2 the detection signal D is monitored. In step S3 it is checked whether the monitored detection signal shows a minimum which indicates that the cell is above the detector (i.e. generally in the middle of the electrode unit). If there is no minimum the drive voltages V are eventually increased in step S1 until the detection signal D shows a minimum.

In step S4, after the detection signal D shows a minimum, the voltage amplitude of said drive voltages are decreased, and again (step S5) the detection signal D is monitored. If an increase of said detection signal is found (step S6) this indicates the moving of the cell away from the detector. As long as no increase is found the voltage amplitude is further decreased (step S4), otherwise (step S7) the drive voltage supplied to one of the electrode elements is switched off and/or the drive voltages supplied to all electrode elements except for one electrode are increased, thus creating a force in the direction of said one electrode element.

In step S8 the detection signal is again monitored to see if there is a decrease of the detection signal, indicating the moving of the cell back to the position of the detector, or an increase of the detection signal indicating the moving of the cell further away from the detector. Steps S7 and S8 are repeated for the other electrode elements by sequentially switching the drive voltage supplied to one of the electrode elements off and/or increasing the drive voltages supplied to all electrode elements except for one electrode and monitoring the detection signal, until an end criterion (e.g. a predetermined number of runs, or steps S7 and S8 have been carried out for all electrode elements) has been met. Then, in step S10 the monitored detection signals are evaluated to get information on the directionality of movement of the cell. This also provides an information whether the directionality is in the direction of the stimulant gradient.

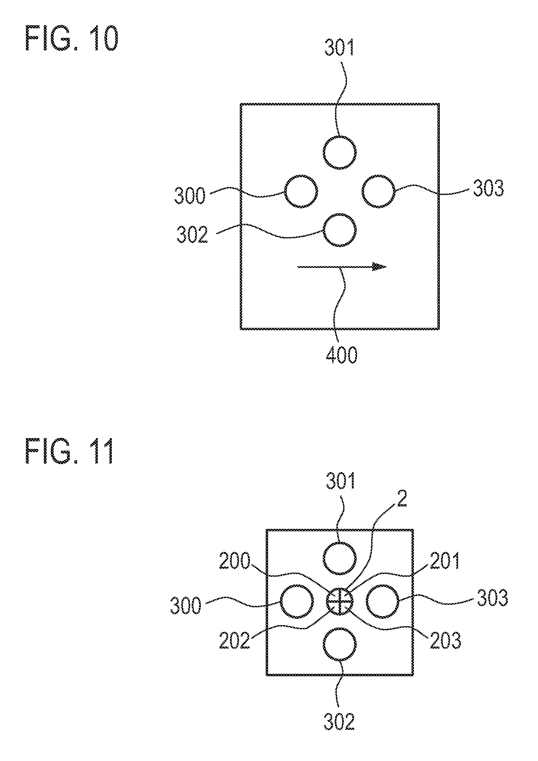

It shall be noted that for measuring the directionality, the stimulant gradient should preferably be along a direction defined by two opposite electrodes (e.g. along the direction 400 defined by the opposite electrodes 300 and 303 as shown in Fig. 10, in order to measure directly the movement parallel and perpendicular to the stimulant gradient.

In the above the electrode elements of an electrode unit are each surrounding only a single optical detector (e.g. as shown in Fig. 3). However, is it also possible that there are more than one optical detector surrounded by the electrodes of a single electrode unit or to use a segmented detector. For instance, as shown in Fig. 11, the electrodes 300 to 303 of a quadrupole electrode surround the optical detector 2 which is segmented into four optical detector segments 200 to 203. This enables measurement of the direction of movement of the cell by monitoring the changes of the detection signals of said optical detector segments 200 to 203.

While the present invention has been described in particular detail, it should also be appreciated that numerous modifications are possible within the intended spirit and scope of the invention. Neither is the invention limited to a particular number of electrode elements within each electrode units, nor to a particular number of optical detectors surrounded by the electrode elements of a particular electrode unit.

The electrode unit can be any configuration of electrodes which creates either a low or a high electrical field region and where the optical detector can be placed in this region. For biological fluids where negative dielectrophoresis is most often observed a low field region is desired. This can be created via the described quadrupoles or other electrode designs such as a ring trap or a segmented ring trap.

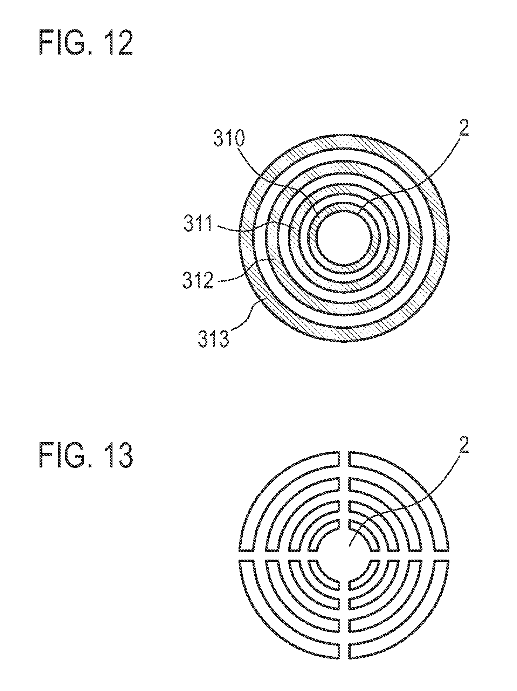

Such a ring trap is schematically shown in Fig. 12, where an optical detector 2 is surrounded by four ring electrodes 310, 311, 312, 313 which are separately provided with drive voltages. If the conductivity is low then sine waves with a 90° phase shift are preferably applied, i.e. 0°, 90°, 180°, 270° to the ring electrodes. If the conductivity is high then a high field region is moved to the middle (i.e. towards the detector 2), sine waves are applied with a phase shift such as 0°, 0°, 0°, 180°, then 0°, 0°, 180°, 0°, and then 0°, 180°, 0°, 0°.

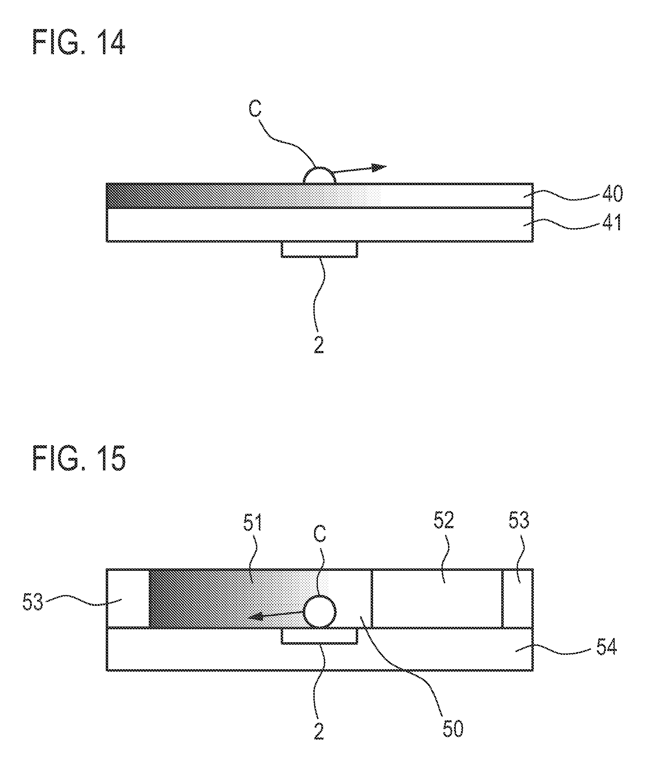

The ring electrodes 310, 311, 312, 313 can also be segmented as shown in Fig. 14, which shows an embodiment in which each ring is segmented into four (equally sized) ring segments. In this embodiment the same method for switching off each electrode is preferably applied as in the quadrupole electrodes described above. The electrode segments of each quadrant are preferably set sequentially, and it is checked which one pushes the cell back to the middle (above the detector 2).

It should be noted that the general idea to have the electrodes on a bottom and/or a top substrate as illustrated in Fig. 8 also holds for the electrodes shown in Figs. 12 and 13.

Fig. 14 shows a schematic diagram of an embodiment using haptotaxis. In haptotaxis the movement of cells is generally induced by a concentration gradient of a substrate-bound stimulus. In this embodiment a collogen layer 40 with cross-linked VEGF (vascular endothelial growth factor, which is an important signaling protein involved in both vasculogenesis and angiogenesis) is provided on top of a carrier layer 41, which carriers the detector 2 on its bottom. The concentration in bound growth factor can be realized in various ways:
i) ink jet printing of growth factor solution where the number of drops (and so also of the concentration) increases;
ii) use of photosensitive gel which binds to growth factor and has a gradient illumination.

The device is generally working in the same way as the above described embodiments for chemotaxis, and also the electrodes (not shown) are placed at the same or similar position and can be adapted in the same manner.

Fig. 15 \ shows a schematic diagram of an embodiment using chemoinvasion. In chemoinvasion the movement of cells into/through a barrier or gel is generally induced by a concentration gradient of a chemotactic stimulus. In this embodiment the stimulant gradient chamber 50 has walls 51 made of gel, which is porous to the stimulant and which separates the stimulant gradient chamber 50 from the neighbouring chamber 52 containing the buffer solution.

The gel should be optically transparent and offer no electrical resistance. Side walls 53 are provided on the outer sides of the chambers 50 and 52. The chambers 50 and 52 and the side walls 53 are provided on a substrate layer 54 (e.g. a glass substrate), which carriers the detector 2 on its top surface. The operation of this embodiment is generally the same as explained above for the embodiments using chemotaxis.

In interpreting the appended claims it should be understood that:
a) the word "comprising" does not exclude the presence of elements other than those listed in a claim; b) the word "a" or "an" preceding an element does not exclude the presence of a plurality of such elements, c) any reference signs in the claims do not limit their scope.

## Claims

1. Device for the monitoring of the movement of cells comprising:
- a stimulant gradient chamber (1) for providing a concentration gradient of a stimulant, said stimulant gradient chamber (1) having a cell inlet (12) for the inlet of cells (C),
- an optical detector (2) for detecting shadows from cells (C) located between a light source and said detector (2) and for generating a detection signal,
- an electrode unit (3) comprising a number of electrode elements surrounding said optical detector (2),
- a voltage supply unit (4) for supplying the electrode elements of said electrode unit (3) with drive voltages,
- a signal measurement unit (5) for measuring said detection signal, and
- a control unit (6) for controlling said voltage supply unit (4) based on said measured detection signal.

2. Device as claimed in claim 1,
wherein said control unit (6) is adapted for controlling the voltage supply of said electrode unit (3) such that, in case of a monitored movement of a cell in a direction away from a holding position, the supplied drive voltage is changed to prevent said movement.

3. Device as claimed in claim 2,
wherein said control unit (6) is adapted for increasing frequency and/or amplitude of the supplied drive voltage in case of a monitored movement of a cell in a direction away from a holding position.

4. Device as claimed in claim 1,
wherein said electrode unit (3) is a quadrupole electrode (30) comprising four electrode elements (300 - 303), and wherein said voltage supply unit (4) is adapted for supplying neighbouring electrode elements (300, 301) with high frequency drive voltages having a phase difference, in particular with drive voltages in a frequency range between 50 kHz and 5 MHz and having a phase difference of 180°.

5. Device as claimed in claim 1,
wherein said electrode unit (3) is an octopole electrode (37, 38) comprising eight electrode elements, wherein four electrode elements (37) are arranged on a first substrate (7) and four electrode elements (38) are arranged on a second substrate (8), which is arranged parallel to the first substrate (7), and wherein said optical detector (2) is arranged in or on the first or second substrate or between the two substrates.

6. Device as claimed in claim 1,
wherein said optical detector (2) is a light sensitive semiconductor element, in particular a PIN diode.

7. Device as claimed in claim 1,
further comprising a light source for illuminating the stimulant gradient chamber (1).

8. Device as claimed in claim 1,
further comprising a calibration unit (14), in particular a photodiode, for calibration of said signal measurement unit (5).

9. Device as claimed in claim 1,
wherein said stimulant gradient chamber (100) includes
- a branch structure (101) having a stimulant inlet (10) for the inlet of a stimulant, a buffer inlet (11) for the inlet of a buffer solution, a plurality of interconnected branches for mixing said stimulant and said buffer solution into said concentration gradient of said stimulant, and a concentration gradient outlet (103) for the outlet of said concentration gradient, and
- a monitoring chamber (102) having said cell inlet (12) and a concentration gradient inlet (104) connected to said concentration gradient outlet (103) for the inlet of said concentration gradient and housing said optical detector (2) and said electrode unit (3).

10. Device as claimed in claim 1,
wherein said stimulant gradient chamber (110) includes
- a stimulant chamber (111) having a stimulant inlet (10) for the inlet of a stimulant,
- a buffer chamber (112) having a buffer inlet (11) for the inlet of a buffer solution,
- a plurality of channels (113) connecting said stimulant chamber (111) and said buffer chamber (112),
- a tapping means (114) for tapping said channels (113) at different tapping locations (115), the distance of said tapping locations (115) from said stimulant chamber (111) being gradually increasing from one channel to the next channel, said tapping means (114) being provided for creating said concentration gradient by tapping at said different tapping locations (113),
- a concentration gradient outlet (116) for the outlet of said concentration gradient, and
- a monitoring chamber (117) having said cell inlet (12) and a concentration gradient inlet (118) for the inlet of said concentration gradient and housing said optical detector (2) and said electrode unit (3).

11. Device as claimed in claim 1,
wherein said stimulant gradient chamber (130) includes a plurality of parallel microfluidic channels (131, 132) located parallel to said electrodes elements (30), each second channel (131) being provided for the inlet of said stimulant and the other channels (132) being provided for the inlet of a buffer solution, wherein the walls (133) of said channels (131, 132) are porous to said stimulant and said buffer solution to allow said stimulant and said buffer solution to diffuse through said walls for creating said concentration gradient between neighbouring channels (131, 132).

12. Device as claimed in claim 11,
wherein said walls (133) are made of a gel type material, in particular a hydrogel.

13. Device as claimed in claim 11,
wherein said stimulant gradient chamber (130) further includes porosity control means (134, 135) for controlling the porosity of the walls (133) of said channels (131, 132).

14. Device as claimed in claim 14,
wherein said porosity control means includes porosity control electrodes (135) arranged close to said walls (133) and wherein said walls (133) are made responsive to an electric voltage applied to said porosity control electrodes or an associated pH change.

15. Device as claimed in claim 1,
wherein said stimulant gradient chamber (120) includes
- a stimulant chamber (121) having a stimulant inlet (10) for the inlet of a stimulant,
- a buffer chamber (122) having a buffer inlet (11) for the inlet of a buffer solution,
- a diffusion chamber (123) connecting said stimulant chamber (121) and said buffer chamber (122) for creating said concentration gradient by diffusion of said stimulant and said buffer solution.

16. Device as claimed in claim 1,
further comprising an adherence layer (9) for adherence of said cells.

17. Device as claimed in claim 16,
wherein said adherence layer (9) is made of a gel type material, in particular a hydrogel or polyacrylamide, and wherein the thickness of said adherence layer (9) is greater than the levitation height of said cells.

18. Device as claimed in claim 16,
wherein said adherence layer (9) covers said optical detector (2) and said electrode unit (3).

19. Device as claimed in claim 16,
wherein said adherence layer (9) is placed on a second substrate (8), which is arranged parallel to a first substrate (7), on which the electrode elements of said electrode unit (3) are arranged.

20. Device as claimed in claims 5 and 16,
wherein said adherence layer (9) is placed on said first and said second substrate (7, 8) covering the electrode elements of said octopole electrodes (37, 38) and wherein said control unit (6) is adapted for controlling the voltage supply of said octopoles (37, 38) such that the electrode elements (37) arranged on the first substrate (7) are provided with drive voltages having a different voltage amplitude and/or frequency than the electrode elements (38) arranged on the second substrate (8).

21. Device as claimed in claim 1,
comprising an array of optical detectors (2) and a plurality of electrode units (3), each including a number of electrode elements and surrounding one optical detector of said array.

22. Device as claimed in claim 1,
comprising at least two optical detectors (200 - 203) surrounded by the electrode elements (300 - 303) of said electrode unit (3).

23. Method for the monitoring of the movement of cells comprising the steps of:
- providing a concentration gradient of a stimulant in a stimulant gradient chamber (1) having a cell inlet (12) for the inlet of cells (C),
- detecting shadows from cells located between a light source and an optical detector (2) and for generating a detection signal from said detection,
- supplying drive voltages to the electrode elements of an electrode unit (3), the electrode elements of an electrode unit surrounding said optical detector (2),
- measuring said detection signal, and
- controlling said voltage supply unit based on said measured detection signal.

24. Method as claimed in claim 23,
wherein movement of a cell is detected by supplying drive voltages having a high frequency and/or high voltage amplitude to said electrode elements thus making the cell move to the position of the detector and wherein thereafter the voltage amplitude and/or frequency of said drive voltages is reduced until movement of the cell is detected.

25. Method as claimed in claim 23,
wherein directionality of the movement of a cell is detected by the steps of
a) applying the same drive voltages to the electrode elements of said electrode unit to move the cell to the position of the detector,
b) monitoring the detection signal and detecting minimum of said detection signal indicating that the cell is located above the detector,
c) decreasing the voltage amplitude of said drive voltages,
d) monitoring the detection signal and detecting an increase of said detection signal indicating the moving of the cell away from the detector,
e) switching the drive voltage supplied to one of the electrode elements off and/or increasing the drive voltage supplied to all electrode elements except for one electrode, thus creating a force in the direction of said one electrode element ,
f) monitoring the detection signal and detecting a decrease indicating the moving of the cell back to the detector or an increase indicating the moving of the cell further away from the detector,
g) repeating steps e) and f) for the other electrode elements by sequentially switching the drive voltage supplied to one of the electrode elements off and/or increasing the drive voltages supplied to all electrode elements except for one electrode and monitoring the detection signal.
